(19) Europäisches Patentamt
European Patent Office
Office européen des brevets

(11) Veröffentlichungsnummer: **0 330 036**
**B1**

(12) # EUROPÄISCHE PATENTSCHRIFT

(45) Veröffentlichungstag der Patentschrift: **26.09.90**

(51) Int. Cl.5: **C07C 45/36,** C07C 47/565, C07C 47/57, C07C 47/575

(21) Anmeldenummer: **89102424.2**

(22) Anmeldetag: **13.02.89**

(54) **Verfahren zur Herstellung von p-Hydroxy-benzaldehyden.**

(30) Priorität: **24.02.88 DE 3805697**

(43) Veröffentlichungstag der Anmeldung: **30.08.89 Patentblatt 89/35**

(45) Bekanntmachung des Hinweises auf die Patenterteilung: **26.09.90 Patentblatt 90/39**

(84) Benannte Vertragsstaaten: **AT BE CH DE FR GB IT LI NL SE**

(56) Entgegenhaltungen:
**EP-A- 0 012 939**
**DE-B- 1 128 847**
**US-A- 3 073 867**
**US-A- 3 666 815**
**US-A- 4 453 016**
**US-A- 4 471 140**

**PATENT ABSTRACTS OF JAPAN, Band 11, Nr. 365 (C-460)[2812], 27. November 1987; & JP-A-62 135 443**

(73) Patentinhaber: **BAYER AG, D-5090 Leverkusen 1 Bayerwerk(DE)**

(72) Erfinder: **Schnatterer, Albert, Dr., Walter-Flex-Strasse 23, D-5090 Leverkusen 1(DE)**
Erfinder: **Fiege, Helmut, Dr., Walter-Flex-Strasse 23, D-5090 Leverkusen 1(DE)**

**EP 0 330 036 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von p-Hydroxy-benzaldehyd und seinen Derivaten durch Oxidation der zugrundeliegenden p-Kresole mit Sauerstoff in Gegenwart von Chelatkomplexen des Eisens und/oder Mangans, wobei als Chelatbildner Porphyrine, Azaporphyrine oder Phthalocyanine eingesetzt werden.

p-Hydroxy-benzaldehyde sind wichtige Zwischenprodukte für industrielle Synthesen von Aromastoffen, Pflanzenschutzprodukten, Farbstoffen und Pharmazeutika.

Die Herstellung von p-Hydroxy-benzaldehyden ist durch Oxidation von p-Kresolen möglich: wobei die selektive Oxidation mit Luftsauerstoff von industriellem Interesse ist.

Nach EP 0 012 939 lassen sich p-Kresolderivate in flüssiger Phase in Gegenwart einer Base und einer Kobaltverbindung oder metallischem Kobalt und einem Alkohol als Lösungsmittel mit Sauerstoff zu p-Hydroxy-benzaldehyden oxidieren. Beispielsweise wird beim Einsatz von 1 Mol-% Kobaltchlorid und 3 Mol an Natriumhydroxid, bezogen auf eingesetztes p-Kresol, eine Selektivität an p-Hydroxy-benzaldehyd von 78 % bei einem Umsatz von 92 % erreicht.

Nach US 4.453.016 wird dieses Verfahren dadurch verbessert, daß ein Salz der Metalle Kobalt, Mangan, Nickel oder Chrom in Kombination mit Aktivkohle eingesetzt wird. Im dort aufgeführten Beispiel wird mit 6,7 Mol-% Kobaltchlorid und 6.7 Gew.-% Aktivkohle, bezogen auf eingesetztes p-Kresol, gearbeitet. Der Zusatz von Aktivkohle erhöht die Reaktionsgeschwindigkeit. In gleicher Weise wirksam ist nach US 4.471.140 auch der Zusatz eines Amins.

Eine Selektivitätsverbesserung gegenüber der durch Kobalt katalysierten Sauerstoffoxidation von p-Kresol zu p-Hydroxy-benzaldehyd wird nach JP 61/24 535 (1986) erreicht, wenn in Gegenwart einer Kombination aus einer Kobalt-und einer Kupferverbindung gearbeitet wird. Durch den Einsatz von zusammen 1,5 Gew.-% Kobalt- und Kupfersalz wird eine Selektivität von 87 % p-Hydroxy-benzaldehyd bei einem p-Kresolumsatz von 97 % erzielt. Nach JP 62/153 240 (1987) kann die Menge der eingesetzten Kobaltverbindung auf ein Zehntel reduziert werden, wenn der Anteil an Eisenionen gleichzeitig unter 6 ppm gehalten wird.

In JP 62/132 836 (1987) wird die Oxidation von p-Kresol zu p-Hydroxy-benzaldehyd in Gegenwart eines Gemisches aus einer Eisen(III)- und einer Nickel(II)-Verbindung durchgeführt und damit ein rascheres Erreichen der Gleichgewichtseinstellung herbeigerührt. Die Selektivitäten liegen bei 52–81% und die Umsätze bei 42–54%. In JP 62/135 443 (1987) wird in Gegenwart von Eisen(III)-Verbindungen, bevorzugt Eisen(III)-Salzen, oxidiert und damit eine Selektivität von 40–60% bei 40–50% Umsetzung erzielt.

Die bisherigen Verfahren haben den Nachteil; daß

a) das katalytisch wirksame Schwermetall zum Teil in beträchtlichen Mengen, meist von ca. 1 Mol-% bezogen auf p-Kresol, eingesetzt wird, wobei bevorzugt wasserlösliche Salze verwendet werden: und

b) die p-Hydroxy-benzaldehyd-Selektivitäten zum Teil nur mäßig sind.

In allen diesen Verfahren gelangen bei der anschließenden wäßrigen Aufarbeitung die Schwermetallsalze in das Abwasser, wodurch dieses Abwasser schwierig zu entsorgen ist und die Katalysatormetalle verloren gehen.

In US 3 666 815 werden Eisenphthalocyanine als Katalisatoren für die Oxidation von sekundären und tertiären Alkylgruppen an Aromaten, beispielsweise für die Cumoloxidation beschrieben. Bei dieser Autoxidation von Alkylaromaten werden Hydroperoxide als Radikalstarter zugesetzt, um eine hohe Reaktionsgeschwindigkeit zu erreichen. Da phenolische Verbindungen wirkungsvolle Radikalfänger sind und autoxidative Prozesse zum Erliegen bringen, ist es überraschend, daß die erfindungsgemäßen Chelatkomplexe bei der hochselektiven Oxidation von p-Methyl-phenolen zu höheren Umsätzen und höhere Reaktionsgeschwindigkeit bei weiter gesteigerter Selektivität führen.

Es wurde nun ein Verfahren zur Herstellung von p-Hydroxy-benzaldehyden der Formel

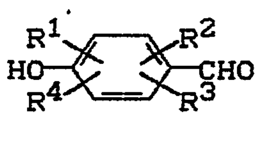

(I),

in der

$R^1$ bis $R^4$ unabhängig voneinander Wasserstoff, Halogen, $C_1$–$C_{10}$-Alkyl, $C_3$–$C_8$-Cycloalkyl, Phenyl oder $C_1$–$C_{10}$-Alkoxy bedeuten,

durch Oxidation von p-Kresolen der Formel

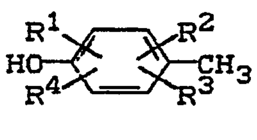

(II),

worin

$R^1$ bis $R^4$ die angegebene Bedeutung haben:

mit Sauerstoff in Gegenwart basisch reagierender Stoffe in einem Lösungsmittel gefunden, das dadurch gekennzeichnet ist, daß in Gegenwart eines Chelatkomplexes von Eisen, Mangan oder Eisen und Mangan gearbeitet wird, wobei als Chelatbildner Porphyrine, Azaporphyrine oder Phthalocyanine eingesetzt werden.

Das erfindungsgemäße Verfahren hat folgende Vorteile:

1. p-Hydroxy-benzaldehyde können aus den zugrundeliegenden p-Kresolen in hoher Selektivität hergestellt werden.
2. die zur Verfahrensdurchführung notwendigen Metallmengen sind deutlich geringer als bei bisherigen Verfahren; und
3. der Katalysator ist rückführbar und stellt damit in ökologischer Hinsicht einen bedeutenden Fortschritt dar.

Erfindungsgemäß einsetzbare p-Kresole sind solche der Formel (I).

Halogen kann Fluor, Chlor, Brom oder Iod sein, bevorzugt Fluor, Chlor oder Brom.

Die Alkylgruppen in $C_1$-$C_{10}$-Alkyl bzw. $C_1$-$C_{10}$-Alkoxy sind beispielsweise Methyl, Ethyl, Propyl, Isopropyl, n-Butyl, i-Butyl, t-Butyl, Hexyl, Octyl oder Decyl. In bevorzugter Weise haben diese Alkylgruppen 1-6 C-Atome, in besonders bevorzugter Weise 1-4 C-Atome.

$C_3$-$C_8$-Cycloalkyl umfaßt Cyclopropyl, Cyclopentyl, Cyclohexyl, Cyclooctyl und deren ein- oder zweifach mit Methyl oder Ethyl substituierten Derivate; bevorzugt sei $C_5$-$C_6$-Cycloalkyl genannt, das durch Methyl substituiert sein kann.

Phenyl kann durch Halogen der genannten Art, durch $C_1$-$C_4$-Alkyl der genannten Art oder durch Nitro ein- oder zweifach substituiert sein.

In bevorzugter Weise werden p-Kresole der Formel

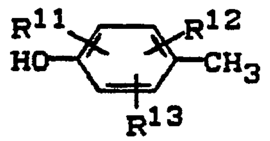

(III)

eingesetzt, worin

$R^{11}$, $R^{12}$ und $R^{13}$ unabhängig voneinander Wasserstoff, Fluor, Chlor, Brom, $C_1$-$C_6$-Alkyl, $C_5$-$C_6$-Cycloalkyl, Phenyl oder $C_1$-$C_6$-Alkoxy bedeuten.

In besonders bevorzugter Weise werden p-Kresole der Formel

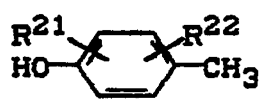

(IV)

eingesetzt, worin

$R^{21}$ für Wasserstoff, Fluor, Chlor, Brom, $C_1$-$C_6$-Alkyl, Cyclohexyl, Phenyl oder $C_1$-$C_6$-Alkoxy steht,

$R^{22}$ Wasserstoff, $C_1$-$C_6$-Alkyl oder $C_1$-$C_6$-Alkoxy bedeutet.

Wichtige, jedoch nur beispielhaft genannte p-Kresole sind:

2-Brom-p-kresol, 3-Chlor-p-kresol, 2,4-Dimethylphenol, 3,4-Dimethylphenol, 2,4,5-Trimethylphenol, 2,4,6-Trimethylphenol, 2,6-Dimethoxy-p-kresol, 2-tert.-Butyl-p-kresol, 2,6-Di-tert.-butyl-p-kresol, 2-Chlor-6-isopropyl-p-kresol. In ganz besonders bevorzugter Weise wird p-Kresol eingesetzt.

Das erfindungsgemäße Verfahren erlaubt die selektive Oxidation nur der zur OH-Gruppe p-ständigen Methylgruppe zur Formylgruppe. Die anderen Substituenten, auch Methylgruppen, bleiben unverändert. Aufgrund dieser Selektivität sind auch Gemische von Kresolen als Ausgangsmaterial geeignet, die neben dem gegebenenfalls substituierten p-Kresol auch o- oder m-Kresole enthalten. Die nicht erfindungsgemäß umgesetzten Isomeren außer dem p-Kresol bleiben im wesentlichen unverändert.

Als Katalysator wird im erfindungsgemäßen Verfahren ein Chelatkomplex des Eisens und/oder Mangans eingesetzt, wobei als Chelatbildner Porphyrine, Azaporphyrine oder Phthalocyanine eingesetzt werden.

Porphyrine sowie damit verwandte, chemisch abgewandelte Systeme (Porphyrinanaloga) sind beispielsweise Corrine, Phlorine, Oxophlorine, Porphyrinogene, Azaporphyrine, Benzoporphyrine, Chlorine.

Die Porphyrine können natürlichen Ursprungs sein, z.B. Haematoporphyrin, Deuteroporphyrin, Protoporphyrin, Uroporphyrin, Koproporphyrin oder rein synthetischer Art, z.B. Octaethylporphyrin. Bevorzugt eingesetzt werden synthetische Porphyrine, besonders bevorzugt meso-Tetraarylporphyrine, z.B. Tetraphenylporphin (TPP), Tetrakis-(4-methoxyphenyl)-porphin, Tetrakis-(2,4-dimethoxyphenyl)-porphin, Tetrakis-(3,4-methylendioxyphenyl)-porphin, Tetrakis-(4-Methylphenyl)-porphin, Tetrakis-(2-

chlorphenyl)-porphin, Tetrakis-(pentafluorphenyl)-porphin.

Im Metallchelatkomplex muß nicht notwendigerweise jede Koordinationsstelle durch einen Chelatliganden besetzt sein. Freie Koordinationsstellen können durch beliebige Donoren besetzt werden, z.B.. durch Anionen anorganischer und organischer Säuren wie $OH^-$, $Cl^-$, $Br^-$, $F^-$, $J^-$, $N_3^-$, $NO_3^-$, $NO_2^-$, $SO_4^{2-}$, $SO_3^-$, Phosphat, Borat, Carboxylat, durch Amine wie Triethylamin, Pyridin, Piperidin, Imidazol, durch Sulfide wie Dimethylsulfid, Dimethylsulfoxid.

Die Chelatkomplexe können eines oder mehrere Metallzentren aufweisen, sie können dimer, oligomer oder polymer sein.

Die Dimerisierung, Oligomerisierung oder Polymerisierung kann durch Brückenbildung zwischen den Metallzentren über Donormoleküle oder durch Vernetzung der Chelatliganden untereinander zustande kommen. Die Metallzentren können z.B. Oxo- oder Peroxo-verbrückt sein. Beispiele für dimere Oxoverbrückte Eisen- und Manganchelatkomplexe sind $(FeTPP)_2O$, $(MnTPP)_2O$, $(FePc)_2O$, $(MnPc)_2O$ (TPP = Tetraphenylporphyrin, Pc = Phthalocyanin). Die Chelatkomplexe können mit einem polymeren Träger vernetzt sein, z.B. mit einem geeignet funktionalisierten Polystyrol.

Der Metallchelatkomplex kann auch in Kombination mit einem oberflächenaktiven Stoff, z.B. Aktivkohle eingesetzt werden.

Das molare Verhältnis von Eisen- und/oder Mangan-Chelatkomplex zu eingesetztem p-Kresol liegt bei 0,000001 - 0,05, vorzugsweise bei 0,00001-0,005, berechnet als Metall. Erstaunlicherweise ist der Eisen- und/oder Mangan-Chelatkomplex bereits in geringsten Mengen hochwirksam. Aus den weiter unten aufgeführten Ausführungsbeispielen geht deutlich hervor, daß der Einsatz einfacher Eisen- bzw. Mangansalze, ebenso wie der Einsatz einfacher Eisen-bzw. Mangankomplexe oder -komplexsalze bei der Sauerstoffoxidation von p-Kresolen zu p-Hydroxy-benzaldehyden im erfindungsgemäßen Verfahren nur vergleichsweise wenig wirksam ist. Unter einfachen Eisen- bzw. Mangansalzen werden verstanden: Die Salze anorganischer und organischer Säuren, hydratisiert wie dehydratisiert, beispielsweise $FeCl_3 \cdot 6\ H_2O$, $FeCl_2 \cdot 4\ H_2O$, $FeSO_4 \cdot 7\ H_2O$, $Fe_2(SO_4)_3 \cdot x\ H_2O$, $Fe(NO_3)_3 \cdot 9\ H_2O$, Eisenphosphate, $Mn(OAc)_2 \cdot 4\ H_2O$, $Mn(OAc)_3 \cdot 2\ H_2O$, $MnCl_2 \cdot 4H_2O$, $MnSO_4 \cdot 4H_2O$, $MnCO_3$, $Mn(NO_3)_2\ x\ 6H_2O$.

Als Beispiele einfacher Eisen- bzw. Mangankomplexe bzw. -komplexsalze, die ebenfalls nur wenig wirksam sind, seien erwähnt $K_3Fe(CN)_6$, $NaFeCN_5NO$, $K_2MnF_6$.

Es ist daher umso erstaunlicher, daß die genannten Chelatkomplexe des Eisens und/oder des Mangans im erfindungsgemäßen Verfahren eine so völlig unerwartet hohe Wirksamkeit und das bereits in kleinsten Konzentrationen entfalten; dies gilt insbesondere für die Eisen- und Manganporphyrine. Bei hohen Umsetzungsgeschwindigkeiten und hohem Umsetzungsgrad werden hohe p-Hydroxy-benzaldehyd-Selektivitäten erreicht; dies gilt insbesondere, wenn in bevorzugter Form der Eisen- und/oder Manganchelatkomplex, insbesondere der entsprechende Porphyrinkomplex in Kombination mit einem der im folgenden genannten Co-Katalysatoren eingesetzt wird.

Solche Co-Katalysatoren, die vorteilhaft aber nicht zwingend notwendig im erfindungsgemäßen Verfahren eingesetzt werden können, sind Verbindungen der Metalle Kupfer, Chrom, Nickel, Silber, Vanadium, Niob, Tantal, Cadmium, Cer oder anderer Lanthanide, wie Neodym oder Praseodym. Es ist weiterhin sehr erstaunlich, daß selbst der Einsatz von Eisen- und/oder Manganverbindungen, obwohl diese Metalle bereits erfindungsgemäß in den Chelatkomplexen vorkommen, zusätzlich zu diesen Chelatkomplexen noch eine Verbesserung des erfindungsgemäßen Verfahrens bewirken. Solche Metallverbindungen können einzeln oder zu mehreren in beliebigen Kombinationen als Co-Katalysatoren zugesetzt werden. Das Metall kann in diesen Verbindungen jede der individuell möglichen Oxidationsstufen einnehmen. Mögliche Einsatzformen solcher Metallverbindungen sind die Metallsalze anorganischer Säuren, beispielsweise die Halogenide, wie Fluoride, Chloride, Bromide oder Iodide, die Sulfate, Nitrate, Carbonate, Phosphate, Borate, Sulfite, Cyanide oder die Salze organischer Säuren, wie die Acetate, Stearate, Oxalate, Citrate, oder Ionenaustauscher, die solche Metalle gebunden enthalten. Es können weiterhin auch Metallkomplexe bzw. Komplexsalze und Metallchelatkomplexe eingesetzt werden. Es ist weiterhin sehr erstaunlich, daß selbst die elementaren Metalle in gleicher Weise wie die Metallverbindungen als Co-Katalysatoren wirksam sind.

In bevorzugter Weise kommen als Co-Katalysator die Metallchelatkomplexe und die Salze anorganischer oder organischer Säuren zur Anwendung. Für den Einsatz als Metallchelatkomplexe gilt das bereits für die obligatorisch einzusetzenden Eisen- und/oder Manganchelatkomplexe Gesagte. Unter den Metallsalzen werden besonders bevorzugt eingesetzt die Chloride, Sulfate, Nitrate, Acetate, Oxide und Hydroxide in hydratisierter oder dehydratisierter Form. Das Metallsalz kann ferner auch in Kombination mit einem komplexierenden Agens, bevorzugt in Kombination mit Ammoniak oder Aminen, eingesetzt werden, beispielsweise Kupferchloride gemeinsam mit Tetramethyl-ethylendiamin.

In besonders bevorzugter Weise werden anorganische Salze von Kupfer und/oder Cer als Co-Katalysator eingesetzt.

Die Einsatzmenge des Co-Katalysators ist keiner spezifischen Beschränkung unterworfen. Auffallend ist die hohe Wirksamkeit bereits in sehr kleinen Konzentrationen. Beispielsweise sei ein molares Verhältnis von Co-Katalysator zu eingesetztem p-Kresol von 0,00001-0,1, vorzugsweise von 0,0001-0,01, genannt. Das molare Verhältnis von Eisen- und/oder Manganchelatkomplex zum Co-Katalysator ist unspezifisch und wird beispielsweise im Bereich 0,001-1000:1, bevorzugt 0,01-100:1, gewählt. Werden

andererseits die als Co-Katalysatoren wirksamen genannten Metallverbindungen einzeln oder in beliebigen Kombinationen ohne die beschriebenen Eisen- und/oder Manganchelatkomplexe eingesetzt, ist ihre Wirksamkeit bei der Oxidation von p-Kresolen vergleichsweise sehr gering.

Als Basen für das erfindungsgemäße Verfahren sind alle geeignet, die eine höhere Basizität aufweisen als die daraus herstellbaren p-Kresolate, beispielsweise Metallhydroxide, Metallalkoholate und Metallamide von Alkali- und Erdalkalimetallen und, soweit es sich um Alkoholate bzw. Amide handelt, von Aluminium. Wichtige Metalle unter den genannten sind beispielsweise Natrium, Kalium, Lithium, Calcium und Magnesium. Als Alkoholate seien das Methylat, das Ethylat, das Isopropylat, das tert.-Butylat aufgeführt. Die Amide können beispielsweise Diethylamid, Ethylamid, Diisopropylamid, Dibutylamid usw. sein. Unter den genannten Basen sind die Hydroxide und die Alkoholate bevorzugt, insbesondere bevorzugt sind Natriumhydroxid, Kaliumhydroxid, Natriummethylat, Natriumethylat und Kalium-tert.-butylat.

Die Base wird in einer Menge von 1-10 Äquivalenten-Base pro Mol des p-Kresols eingesetzt; in bevorzugter Weise werden 1,5-6 Äquivalenten-Base eingesetzt.

In der Praxis erweist sich der Einsatz eines Basengemisches aus einem der genannten Alkali- oder Erdalkalihydroxide und einem der genannten Alkalimetallalkoholate als besonders günstig. Bevorzugte Basengemische sind solche aus einem Alkalihydroxid und einem Alkalialkoholat des Methanols oder Ethanols. Im besonderen bevorzugt sind Gemische aus Natrium- oder Kaliumhydroxid und Natriummethanolat. Die anteilmäßige Zusammensetzung solcher Basengemische aus Alkali- oder Erdalkalihydroxid und Alkalimetallalkoholat, welche einen Vorteil gegenüber dem Arbeiten mit einerseits ausschließlich Alkali- oder Erdalkalihydroxid und andererseits ausschließlich Alkalialkoholat bieten, kann in dem weiten Bereich von 1:50 - 50:1 schwanken. Bevorzugt wird bei einem Äquivalentverhältnis von Alkalihydroxid zu Alkalialkoholat von 8:1 - 1:4, besonders bevorzugt 4:1 - 2:3 gearbeitet.

Lösungsmittel für das erfindungsgemäße Verfahren sind solche, die unter den Reaktionsbedingungen nicht oder nur sehr langsam mit Sauerstoff reagieren und die Ausgangskomponenten lösen, beispielsweise Alkohole, Ether, halogenierte Kohlenwasserstoffe, Amine, Amide, Sulfoxide. Solche Lösungsmittel können einzeln oder in Gemischen von zwei oder mehreren eingesetzt werden. Sofern diese Lösungsmittel ganz oder teilweise mit Wasser mischbar sind, kommen auch solche Mischungen mit Wasser in Frage. Die alkoholischen Lösungsmittel sind bevorzugt, beispielsweise Methanol, Ethanol, Isopropanol, Butanol, tert.-Butanol und/oder Ethylenglykol.

Für den Fall der Verwendung von Alkoholen als Lösungsmittel findet man im Reaktionsgemisch gegebenenfalls substituierte p-Hydroxy-benzylalkylether der Formel

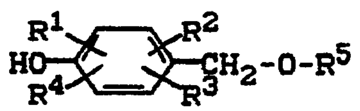

(V),

worin

$R^1$ bis $R^4$ den oben angegebenen Bedeutungsumfang haben und

$R^5$ der Alkylrest des als Lösungsmittel verwendeten Alkohols ist.

Die Menge dieser Ether schwankt mit den Reaktionsbedingungen.

Als Sauerstoff für das erfindungsgemäße Verfahren kann reiner Sauerstoff oder Sauerstoff in verdünnter Form, beispielsweise in Form von Sauerstoff enthaltenden Gasen eingesetzt werden. Die wirtschaftlich günstigste Form des erfindungsgemäß verwendbaren Sauerstoffs ist die atmosphärische Luft. Der Druck des Sauerstoffs bzw. des Sauerstoff enthaltenden Gases ist keiner besonderen Einschränkung unterworfen und kann zwischen 1-100 bar liegen, vorzugsweise bei 1-10 bar. Der Sauerstoffgehalt ist bei Verwendung von Sauerstoff enthaltenden Gasen ebenfalls keiner Beschränkung unterworfen. Er richtet sich in erster Linie nach betrieblichen Gesichtspunkten, wie der Betriebssicherheit und der Umsetzungsgeschwindigkeit. Der Sauerstoff kann beispielsweise in das Reaktionsmedium eingespeist werden, beispielsweise unter Verwendung von Fritten; er kann jedoch auch durch kräftiges Rühren in das Reaktionsgemisch eingezogen werden.

Die Reaktionstemperatur kann in weiten Grenzen schwanken, beispielsweise von 0-200°C, bevorzugt 20-100°C.

Bei der Aufarbeitung im Anschluß an das erfindugsgemäße Verfahren werden zunächst die Katalysatoren vom reaktionsgemisch abgetrennt (beispielsweise durch Filtration oder Zentrifugieren) und gegebenenfalls wieder eingesetzt; des weiteren wird nicht umgesetztes p-Kresol und der Teil des Reaktionsgemisches, der nur bis zur Stufe des p-Hydroxy-benzylalkylethers oxidiert wurde, in einen Folgeansatz zurückgeführt.

Die Art der Aufarbeitung richtet sich nach den Ausführungsbedingungen. Ist beispielsweise der Metallchelatkomplex im verwendeten Lösungsmittel schwer löslich, wie etwa Eisentetraphenylporphin in Methanol, kann er zusammen mit dem Co-Katalysator, welcher bei entsprechender Einsatzform, beispielsweise als Metallsalz, anschließend als unlösliches Oxidhydrat vorliegt, direkt vom Reaktionsgemisch abfiltriert werden, wobei dieses bei Bedarf genügend verdünnt wurde, um Einsatzstoffe und Produkte zu lösen. Das Filtrat kann anschließend eingedampft, in Wasser aufgenommen und der p-Hydroxy-benzaldehyd durch Ansäuern freigesetzt werden.

Ist der Metallchelatkomplex im verwendeten Lösungsmittel gut löslich, wie beispielsweise Mangantetraphenylporphin in Methanol, in Wasser aber unlöslich, so kann das Lösungsmittel aus dem Reaktionsgemisch abgedampft, der Rückstand in Wasser aufgenommen und der Metallchelatkomplex zusammen mit dem als unlöslichem Oxidhydrat vorliegendem Co-Katalysator abfiltriert werden. Da Produkte und Einsatzstoffe in Form ihrer Salze (Phenolate) vorliegen, sind sie in Wasser ausreichend löslich. Aus dem Filtrat kann durch Ansäuern wiederum der p-Hydroxy-benzaldehyd freigesetzt werden.

In einer noch weiteren Variante kann das Reaktionsgemisch direkt mit Wasser versetzt werden (vorausgesetzt, es besteht ausreichende Mischbarkeit mit dem Lösungsmittel) und der wasserunlösliche Metallchelatkomplex zusammen mit dem als Oxidhydrat vorliegenden Co-Katalysator abfiltriert werden. Aus dem Filtrat wird das organische Lösungsmittel beispielsweise destillativ abgetrennt, und aus der wäßrig-alkalischen Lösung wird der p-Hydroxy-benzaldehyd durch Ansäuern freigesetzt.

Als Beispiele für besonders vorteilhafte Ausführungsformen seien genannt: Methanol als Lösungsmittel, Natriumhydroxid, Natriummethanolat, Kaliumhydroxid oder Gemische aus diesen als Base, Eisentetraphenylporphin als Metallchelatkomplex und eine Kupferverbindung, wie $CuCl_2 \cdot 2 H_2O$ oder CuO, als Co-Katalysator.

Eine bevorzugte Aufarbeitungsvariante besteht darin, daß man das Reaktionsgemisch durch Versprühen trocknet, die löslichen Bestandteile des trockenen Rückstandes in heißem Wasser löst und sodann das Eisentetraphenylporphin und das unlösliche Kupferoxidhydrat abfiltriert und gegebenenfalls recyclisiert. Der Reaktionsmischung werden dabei im allgemeinen 2-50, vorzugsweise 3-20 Gew.-Teile Wasser pro 1 Gew.-Teil zur Oxidation eingesetztes p-Kresol zugefügt. Für den Fall, daß das Natrium- oder Kaliumsalz das p-Hydroxy-benzaldehyds in kaltem Wasser schlecht löslich ist, wird als Lösung auf 50–90°C erwärmt und bei dieser Temperatur filtriert. In einer Variante dieser Aufarbeitungsform kann das Wasser direkt zum Reaktionsgemisch gegeben werden, die entstehende Lösung auf 50–90°C erwärmt werden und das Eisentetraphenylporphin zusammen mit dem Oxidhydrat des Kupfers abfiltriert werden.

In einer noch weiteren Aufarbeitungsvariante wird das Reaktionsgemisch mit Methanol auf das 1,5-5-fache des Volumens verdünnt, die Lösung erwärmt, vorzugsweise auf 40–65°C, und das schwerlösliche Eisentetraphenylporphin zusammen mit dem unlöslichen Oxidhydrat des Kupfers abfiltriert. Sodann wird das Filtrat durch Versprühen getrocknet und der Rückstand in Wasser aufgenommen.

Alle hier aufgeführten Aufarbeitungsvarianten können dahingehend geändert werden, daß vor dem Abfiltrieren des Eisen- und/oder Manganchelatkomplexes der genannten Art und der als Metalloxidhydrate vorliegenden Co-Katalysatoren der pH-Wert der stark alkalischen Lösung durch Ansäuern auf einen Wert zwischen 9–13 eingestellt wird. Durch diese Maßnahme läßt sich die Löslichkeit der Metallverbindungen gegebenenfalls herabsetzen.

Aus der wäßrig-alkalischen Lösung werden durch Ansäuern der p-Hydroxy-benzaldehyd und, soweit vorhanden, p-Hydroxy-benzylalkylether und nicht umgesetztes p-Kresol freigesetzt. Die Abtrennung und Reinigung des p-Hydroxy-benzaldehyds ist nach den bekannten Methoden, beispielsweise durch fraktionierte Kristallisation, gegebenenfalls unter Zuhilfenahme der Methode der Bisulfit-Adduktbildung möglich.

Sehr reiner p-Hydroxybenzaldehyd läßt sich durch Extrahieren des Rohprodukts mit wäßriger Bisulfitlösung und anschließendes Freisetzen des p-Hydroxybenzaldehyds durch Ansäuern gewinnen.

Die Abtrennung und Reinigung des p-Hydroxybenzaldehyds gelingt in Verbindung mit dem erfindungsgemäßen Verfahren besonders einfach, wenn in die bei dem erfindungsgemäßen Verfahren nach dem Abfiltrieren der Katalysatoren anfallende alkalische Lösung direkt $SO_2$ eingeleitet wird, beispielsweise bis die Lösung pH 2 erreicht hat. Durch Extraktion mit einem geeigneten organischen Lösungsmittel lassen sich dann nicht umgesetztes p-Kresol oder im Falle, daß Kresolgemische eingesetzt wurden, die nicht umgesetzten o- und m-Isomeren, sowie der p-Hydroxybenzylmethylether und Verharzungsprodukte abtrennen. Aus der wäßrigen Lösung wird anschließend durch Ansäuern und Erwärmen das $SO_2$ ausgetrieben und der p-Hydroxybenzaldehyd freigesetzt.

Beispiel 1

Ein Gemisch aus 10,8 g (0,1 Mol) p-Kresol, 12,0 g (0,30 Mol) Natriumhydroxid, 25 g Methanol und 0,10 g (0.14 mmol) Eisentetraphenylporphin (als FeTPPCl) wurde in einer Sauerstoffatmosphäre unter Normaldruck bei 60°C 2,5 Stunden gerührt.

Anschließend wurde das Methanol unter vermindertem Druck abdestilliert, der Rückstand in 70 ml Wasser aufgenommen, auf 70°C erwärmt und filtriert. Von dem Filter konnten nach dem Trocknen 0,092 g Eisentetraphenylporphin zurückgewonnen werden. Das Filtrat wurde mit verdünnter Schwefelsäure angesäuert und mit Ethylacetat extrahiert. Nach Abdampfen des Ethylacetats im Vakuum verblieben 11,04 g Produkt mit einem Gehalt an (ermittelt durch HPLC = Hochdruckflüssigkeitschromatographie) 9,0 % Kresol, 55,5 % p-Hydroxybenzaldehyd, 5,3 % p-Hydroxybenzylmethylether. Das entspricht einem Kresol-Umsatz von 90, 8 %, einer p-Hydroxybenzaldehyd-Selektivität von 55,3 % und einer p-Hydroxybenzylmethylether-Selektivität von 4,7 %.

Beispiel 2

Durchführung wie Beispiel 1 mit dem Unterschied, daß 1,0 g Aktivkohle zugesetzt wurde. Die Umsetzung wurde nach 4 Stunden abgebrochen und das Reaktionsgemisch wie im Beispiel 1 aufgearbeitet. Man erhielt 10,36 g Rohprodukt mit einem Gehalt an 80,1 % p-Hydroxybenzaldehyd, 7,2 % p-Hydroxybenzylmethylether, 2,0 % p-Kresol. Daraus folgt für die p-Hydroxybenzaldehyd-Selektivität 69.3 %, für die p-Hydroxybenzylmethylether-Selektivität 5,5 % bei einer Umsetzung des p-Kresols von 98,1 %.

Beispiel 3

Ein Gemisch aus 10,8 g (0,10 Mol) p-Kresol, 12,0 g (0,30 Mol) Natriumhydroxid, 25 g Methanol, 0,10 g (0,14 mmol) Eisentetraphenylporphin (als FeTPPCl) und 0,043 g (0,25 mmol) $CuCl_2 \cdot 2 H_2O$ wurden in einer Sauerstoffatmosphäre unter Normaldruck bei 60°C 7 Stunden gerührt.

Das Reaktionsgemisch wurde wie in Beispiel 1 aufgearbeitet und brachte folgendes Ergebnis:
Umsetzung des p-Kresols: 96,4%
p-Hydroxybenzaldehyd-Selektivität: 78,2 %
p-Hydroxybenzylmethylether-Selektivität: 3,4 %

Beispiel 4

Ein Gemisch aus 10,8 g (0,10 Mol) p-Kresol, 12,0 g (0,30 Mol) Natriumhydroxid, 25 g Methanol, 0,10 g (0,14 mmol) Eisentetraphenylporphin (als FeTPPCl), 0,043 g (0,25 mmol) $CuCl_2 \cdot 2 H_2O$ und 0,137 g (0,25 mmol) $(NH_4)_2 Ce(NO_3)_6$ wurden in einer Sauerstoffatmosphäre unter Normaldruck bei 60°C 7 Stunden gerührt.

Das Reaktionsgemisch wurde wie in Beispiel 1 aufgearbeitet.
Ergebnis: Umsetzung des p-Kresols: 98,8 %
p-Hydroxybenzaldehyd-Selektivität: 82,0 %
p-Hydroxybenzylmethylether-Selektivität: 0,3 %

Beispiel 5

Reaktionsansatz und Durchführung wie in Beispiel 3. Die Abtrennung und Reinigung des p-Hydroxybenzaldehyds erfolgt über das Bisulfit-Addukt in der nachstehend beschriebenen Weise:
Nach Beendigung der Sauerstoffeinleitung wurde das Methanol vom Reaktionsgemisch abdestilliert, der Rückstand in 70 ml Wasser aufgenommen, auf 70°C erwärmt und filtriert. Auf dem Filter verblieben nach Auswaschen mit Wasser und Trocknen 0,115 g Katalysatorgemisch, bestehend aus Eisentetraphenylporphin und Kupferoxidhydrat. In das alkalische Filtrat wurde anschließend bei Raumtemperatur $SO_2$ eingeleitet, bis pH=2 erreicht war. Danach wurde noch 1 Stunde gerührt und dann zweimal mit Ethylacetat ausgeschüttelt. Die resultierende organische Phase wurde über Natriumsulfat getrocknet und das Lösungsmittel abdestilliert. Es blieben 2,31 g Öl als Rückstand mit der Zusammensetzung (Ausbeute)
p-Kresol 2,8 % (0,6 %)
p-Hydroxybenzaldehyd 11,7 % (2,2 %)
p-Hydroxybenzylmethylether 32,1 % (5,4 %)

Die wäßrige Phase wurde mit 50 ml konz. HCl angesäuert und das $SO_2$ durch Erwärmen und durch Anlegen eines leichten Vakuums möglichst vollständig entfernt. Der freigesetzte p-Hydroxybenzaldehyd wurde aus der wäßrigen Phase durch dreimaliges Ausschütteln mit Ethylacetat extrahiert, die organische Phase durch Waschen mit Hydrogencarbonatlösung entsäuert, über Natriumsulfat getrocknet und eingedampft.Als Rückstand fielen 8,74 g farbloses feinkristallines Produkt mit einem Gehalt von 99,4 % p-Hydroxybenzaldehyd an (Ausbeute 71,2 %).

Beispiele 6-16

Ein Gemisch aus 10,8 g (0,10 Mol) p-Kresol, 12,0 g (0,30 Mol) Natriumhydroxid, 25 g Methanol, 0,10 g (0,14 mmol) Eisentetraphenylporphin (als FeTPPCl) und 0,25 mmol der in Tabelle 1 aufgeführten Metallverbindungen wurden in einer Sauerstoffatmosphäre unter Normaldruck bei 60°C innerhalb der ebenfalls in Tabelle 1 angegebenen Zeit intensiv gerührt. Das Reaktionsgemisch wurde wie in Beispiel 1 aufgearbeitet und mittels HPLC analysiert. Die Tabelle 1 zeigt die Ergebnisse hinsichtlich der Umsetzung des p-Kresols und der Selektivitäten in der Bildung des p-Hydroxybenzaldehyds (p-HBA) und des p-Hydroxybenzylmethylethers (p-HBME).

**Tabelle 1**

| Beispiel Nr. | Metallverbindung | Reaktionszeit (h) | Umsetzung % | Selektivität % p-HBA | Selektivität % p-HBME |
|---|---|---|---|---|---|
| 6 | $(NH_4)\cdot Ce(NO_3)_6$ | 6 | 96,2 | 74,6 | 2,8 |
| 7 | $Mn(OAc)_2\cdot 4\ H_2O$ * | 7 | 91,1 | 62,9 | 11,7 |
| 8 | $Cr(NO_3)_3\cdot 9\ H_2O$ | 7 | 88,1 | 57,5 | 12,2 |
| 9 | $FeCl_2\cdot 4\ H_2O$ | 7 | 91,6 | 61,8 | 15,5 |
| 10 | $Ni(NO_3)_2\cdot 6\ H_2O$ | 7 | 96,0 | 58,4 | 17,2 |
| 11 | VO(acac) * | 7 | 96,9 | 68,7 | 11,9 |
| 12 | AgOAc * | 7 | 94,8 | 53,3 | 17,8 |
| 13 | $NbCl_5$ | 7 | 98,6 | 63,9 | 3,1 |
| 14 | $TaCl_5$ | 7 | 99,4 | 70,2 | 11,6 |
| 15 | $Cd(NO_3)_2\cdot 4\ H_2O$ | 7 | 95,1 | 61,2 | 12,1 |
| 16 | $Nd(NO_3)_2\cdot 6\ H_2O + Pr(NO_3)_3\cdot 6\ H_2O$ (7:3) | 7 | 94,9 | 62,1 | 18,6 |

*Ac = Acetyl, acac = Rest von Acetyl-acetonat

Beispiele 17 bis 25 (Vergleichsbeispiele)

In den Beispielen 17 bis 25 wurde zum Vergleich die Wirksamkeit einfacher Eisen- und Mangansalze sowie die Wirksamkeit einiger als Co-Katalysator eingesetzter Metallverbindungen ohne Zusatz von Eisentetraphenylporphin, jedoch unter sonst gleichen Bedingungen wie in den vorangegangenen Beispielen, bei der Sauerstoffoxidation von p-Kresol getestet.

Ein Gemisch aus 10,8 g (0,10 Mol) p-Kresol, 12,0 g (0,30 Mol) Natriumhydroxid, 25 g Methanol und dem in Tabelle 2 genannten Metallsalz in der dort angegebenen Menge wurde in einer Sauerstoffatmosphäre unter Normaldruck bei 60°C innerhalb 7 Stunden intensiv gerührt. Die Ergebnisse hinsichtlich der Umsetzug des p-Kresols und der Selektivitäten in der Bildung des p-Hydroxybenzaldehyds (p-HBA) und des p-Hydroxybenzylmethylethers (p-HBME) sind in Tabelle 2 zusammengefaßt.

**Tabelle 2**

| Beispiel Nr. | Metallverbindung (zugesetzte Menge) | Umsetzung % | Selektivität % p-HBA | Selektivität % p-HBME |
|---|---|---|---|---|
| 17 | $FeSO_4 \cdot 7\ H_2O$ (2,5 mmol) | 25,7 | 41,8 | 7,0 |
| 18 | $K_3Fe(CN)_6$ (2,5 mmol) | 31,5 | 2,2 | 0,7 |
| 19 | $FeCl_3 \cdot 6\ H_2O$ (1 mmol) | 33,9 | 51,2 | 1,5 |
| 20 | $FeCl_3 \cdot 6\ H_2O$ (5 mmol) | 32,0 | 48,0 | 6,4 |
| 21 | $Ni(NO_3)_2 \cdot 6\ H_2O$ (1,0 mmol) | 10,9 | 7,2 | 2,5 |
| 22 | $Mn(OAc)_2 \cdot 4\ H_2O$ (1,0 mmol) | 16,2 | 9,2 | - |
| 23 | $Cr(NO_3)_3 \cdot 9\ H_2O$ (1,0 mmol) | 8,3 | 10,1 | - |
| 24 | $FeCl_3 \cdot 6\ H_2O$ (1,0 mmol) + $Ni(NO_3)_2 \cdot 6\ H_2O$ (1,0 mmol) | 33,3 | 48,9 | 0,4 |
| 25 | $CuCl_2 \cdot 2\ H_2O$ (1,0 mmol) | 33,9 | 12,1 | 4,7 |

Beispiel 26

Katalysatorrückführung: Ein Gemisch aus 10,8 g (0,10 Mol) p-Kresol, 12,0 g (0,30 Mol) Natriumhydroxid, 25 g Methanol, 0,30 g (0,43 mmol) Eisentetraphenylporphin (als FeTPPCl) und 0,128 g (0,75 mmol) $CuCl_2 \cdot 2\ H_2O$ wurde in einer Sauerstoffatmosphäre unter Normaldruck bei 60°C 6 Stunden gerührt. Anschließend wurde das Methanol unter vermindertem Druck abdestilliert, der Rückstand in 70 ml Wasser aufgenommen, auf 70°C erwärmt und filtriert. Der Filterrückstand wurde zweimal mit je 15 ml heißem Wasser ausgewaschen, getrocknet und bei der nächsten Oxidation, die auf dieselbe Weise durchgeführt wurde mit dem Unterschied, daß weder weiteres Eisentetraphenylporphin noch weiteres Kupferchlorid (auch nicht ergänzend mit der Ausnahme der 6. Rückführung) eingesetzt wurde, zum Ausgangsgemisch hinzugefügt. Die Aufarbeitung erfolgte wie im Beispiel 1. In der Tabelle 3 sind die Ergebnisse der Katalysatorrückführungen zusammengestellt. Bei der 6. Rückführung wurde ergänzend nochmals 0,04 g $CuCl_2 \cdot 2\ H_2O$ hinzugefügt. Nach der 10. Rückführung betrug die Katalysatormenge noch 0,14 g. Daraus konnten 0,11 g reines Tetraphenylporphin isoliert werden.

Tabelle 3

| | Umsetzung % | Selektivität % P-HBA | Selektivität % p-HBME |
|---|---|---|---|
| Beispiel 26 | 91,2 | 56,7 | 2,4 |
| Rückführung 1 | 96,2 | 64,2 | 15,76 |
| 2 | 94,5 | 63,6 | 9,7 |
| 3 | 91,7 | 67,0 | 10,7 |
| 4 | 95,4 | 64,5 | 16,7 |
| 5 | 95,7 | 59,3 | 7,4 |
| 6 | 88,8 | 62,4 | 10,4 |
| 7 | 95,9 | 60,5 | 9,0 |
| 8 | 92,0 | 66,4 | 13,9 |
| 9 | 90,5 | 61,9 | 3,2 |
| 10 | 89,6 | 73,9 | 0,2 |

Beispiele 27 bis 31

Ein Gemisch aus 10,8 g (0,10 Mol) p-Kresol, 12,0 g (0,30 Mol) Natriumhydroxid, 25 g Methanol und dem in Tabelle 5 genannten Metallkomplex in der dort angegebenen Menge wurde in einer Sauerstoffatmosphäre unter Normaldruck bei 60°C 5-7 Stunden gerührt. Das Reaktionsgemisch wurde wie in Beispiel 1 aufgearbeitet. Zu den Ergebnissen siehe Tabelle 4.

Tabelle 4

| Beispiel Nr. | Metallverbindung (zugesetzte Menge in mmol) | Reaktionszeit (h) | Umsetzung % | Selektivität % p-HBA | Selektivität % p-HBME |
|---|---|---|---|---|---|
| 27 | MnTPPCl* (0,15 mmol) | 5 | 48,8 | 62,8 | 6,4 |
| 28 | $(FeTPP)_2O$** (0,15 Mol) | 6 | 92,5 | 61,2 | 5,3 |
| 29 | Eisenphthalocyanin (0,50 mmol) | 6 | 35,4 | 42,5 | 6,9 |
| 30 | Eisenporphyrazin (0,30 mmol) | 7 | 33,7 | 64,6 | 15,5 |
| 31 | Hämin (0,30 mmol) | 7 | 56,1 | 48,5 | 3,9 |

* Mangan-tetraphenylporphin mit Ladungsausgleich durch $Cl^-$

** Sauerstoff-verbrücktes dimeres Eisenporphyrin; Eisen-tetraphenylporphin mit Ladungsausgleich durch $Cl^-$ wurde mit NaOH behandelt, wobei sich die dimere Form mit Sauerstoffbrücke bildete.

Beispiel 32

Zu einem m/p-Kresolgemisch, bestehend aus 10,8 g (0,10 Mol) p-Kresol und 10,8 g (0,10 Mol) m-Kresol, wurden 50 g Methanol, 24,0 g (0,60 Mol) Natriumhydroxid, 0,20 g (0,28 mmol) Eisentetraphenylporphin (als FeTPPCl) und 0,085 g (0,50 mmol) $CuCl_2 \cdot 2 H_2O$ gegeben und das Ganze in einer Sauerstoffatmosphäre bei 60°C unter Normaldruck während 7 Stunden intensiv gerührt. Das Reaktionsgemisch wurde wie in Beispiel 1 aufgearbeitet. Die Analyse brachte folgendes Ergebnis:
Umsetzung des p-Kresols: 98,0 %
p-Hydroxybenzaldehyd-Selektivität: 75,1 %
p-Hydroxybenzylmethylether-Selektivität: 13,8 %
Rückgewinnung des m-Kresols: 87,6 %
Das Reaktionsgemisch enthielt weder 3-Hydroxybenzaldehyd noch 3-Hydroxybenzylmethylether.

Beispiele 33 bis 36

Von den in Tabelle 5 aufgeführten p-Kresolderivaten wurden jeweils 0,10 Mol zusammen mit 12,0 g (0,30 Mol) Natriumhydroxid, 50 g Methanol, 0,10 g (0,14 mmol) Eisentetraphenylporphin (als FeTPPCl) und 0,043 g (0,25 mmol) $CuCl_2 \cdot 2 H_2O$ in einer Sauerstoffatmosphäre bei 60°C unter Normaldruck innerhalb der in Tabelle 5 angegebenen Zeit intensiv gerührt. Die Reaktionsgemische wurden analog Beispiel 1 aufgearbeitet und die Produkte analysiert. Der Umsetzungsgrad der entsprechenden p-Kresole sowie die Selektivitäten bezüglich der Bildung der 4-Hydroxybenzaldehydderivate sind in der Tabelle 5 zusammengestellt.

Tabelle 5

| Beispiel Nr. | p-Kresolderivat | Reaktionszeit (h) | 4-Hydroxybenzaldehydderivat | Selektivität (%) | Umsatz (%) |
|---|---|---|---|---|---|
| 33 | 3-Chlor-p-kresol | 5 | 2-Chlor-4-hydroxybenzaldehyd | 72,7 | 95 |
| 34 | 2-Brom-p-kresol | 3 | 3-Brom-4-hydroxybenzaldehyd | 83,1 | 86 |
| 35 | 2-Chlor-6-isopropyl-p-kresol | 5 | 3-Chlor-5-isopropyl-4-hydroxybenzaldehyd | 67,2 | 98 |
| 36 | 2,6-Di-t-butyl-p-kresol | 4 | 3,5-Di-tert.-butyl-4-hydroxybenzaldehyd | 52,1 | 97 |

Die nachstehend aufgeführten Beispiele 37-47 wurden in einem größeren Reaktor bei anteilmäßig höheren Methanol- und Basenmengen, verglichen mit den Beispielen 1-36, durchgeführt.

Beispiel 37

In einem 1 l-Reaktor wurde in ein auf 60°C erwärmtes Gemisch aus 54,0 g (0,50 Mol) p-Kresol, 250 g Methanol, 100 g (2,5 Mol) Natriumhydroxid, 0,25 g (0,35 mmol) Eisentetraphenylporphin (als FeTPPCl) und 0,10 g (1,3 mmol) Kupfer(II)oxid während 7 Stunden unter kräftigem Rühren ein Sauerstoffstrom von 5,0 l/h geleitet. Während der gesamten Reaktionszeit wurde die Temperatur bei 60°C gehalten.

Nach Beendigung der Reaktion wurde das Methanol unter vermindertem Druck abdestilliert, der Rückstand in 700 ml Wasser aufgenommen, mit konz. Salzsäure auf pH = 11,5 eingestellt, dann auf 70°C erwärmt und filtriert. Das Filtrat wurde nach dem Abkühlen auf Raumtemperatur mit konz. Salzsäure angesäuert und anschließend mit Ethylacetat extrahiert. Nach Abdampfen des Ethylacetats im Vakuum verblieben 60,8 g Rohprodukt mit einem Gehalt von 76,9 % p-Hydroxybenzaldehyd, 1,9 % p-Hydroxybenzylmethylether und 0,4 % p-Kresol.

Ausbeute an p-Hydroxybenzaldehyd: 76,6 %.

Beispiel 38

Durchführung wie Beispiel 37 mit dem Unterschied, daß anstelle des Natriumhydroxids 138 g (2,5 Mol) Natriummethanolat (98 %) eingesetzt wurden.

Umsatz >99 %

Ausbeute:

70,4% p-Hydroxybenzaldehyd

0,5% p-Hydroxybenzylmethylether

Beispiel 39

In einem 1 l-Reaktor wurde in ein Gemisch aus 81 g (0,75 Mol) p-Kresol, 250 g Methanol, 60 g (1,5 Mol) Natriumhydroxid, 54 g (1,0 Mol) Natriummethanolat, 0,375 g (0,53 mmol) Eisentetraphenylporphin (als FeTPPCl) und 0,15 g (1,9 mmol) Kupfer(II)oxid bei 60°C während 8 Stunden unter kräftigem Rühren ein Sauerstoffstrom von 5,0 l/h geleitet.

Aufarbeitung wie in Beispiel 37.

Umsatz >99 %

Ausbeute:

85,3 % p-Hydroxybenzaldehyd

2,6% p-Hydroxybenzylmethylether

Beispiel 40

Durchführung wie Beispiel 39 mit dem Unterschied, daß die Menge an Eisentetraphenylporphin auf 0,075 g (0,11 mmol) reduziert wurde.

Umsatz >99 %

Ausbeute:

86,1 % p-Hydroxybenzaldehyd

1,7 % p-Hydroxybenzylmethylether

Beispiele 41-47

In einem 1 l-Reaktor wurde in ein Gemisch aus 54,0 g (0,50 Mol) p-Kresol, 250 g Methanol, 50 g (1,25 Mol) Natriumhydroxid, 54 g (1,0 Mol) Natriummethanolat, 0,10 g (1,3 mmol) Kupfer(II)oxid und 0,07 mmol Eisentetraarylporphin (als FeTAPCl = Eisentetraarylporphin mit Ladungsausgleich durch $Cl^-$; TAP = Tetraarylphorphin) mit den in der Tabelle 6 aufgeführten Tetraarylporphin-systemen bei 60°C während 7 Stunden unter kräftigem Rühren ein Sauerstoffstrom von 4,0 l/h eingeleitet. Die Ergebnisse sind in der Tabelle 6 zusammengefaßt. Der p-Kresolumsatz war jeweils >99 %.

**Tabelle 6**

| Beispiel Nr. | Tetraarylporphin | Ausbeute % p-HBA | Ausbeute % p-HBME |
|---|---|---|---|
| 41 | Tetrakis-(4-methoxyphenyl)porphin | 92,9 | 0,7 |
| 42 | Tetrakis-(3,4-methylendioxy-phenyl)porphin | 88,4 | 1,4 |
| 43 | Tetrakis-(4-methylphenyl)porphin | 87,7 | 1,1 |
| 44 | Tetrakis-(4-chlorphenyl)porphin | 76,2 | 6,8 |
| 45 | Tetrakis-(2-chlorphenyl)porphin | 83,5 | 0,7 |
| 46 | Tetrakis-(4-t-butylphenyl)porphin | 81,3 | 1,9 |
| 47 | Tetrakis-(2,4-dimethoxyphenyl)porphin | 90,6 | 0,6 |

## Patentansprüche

1. Verfahren zur Herstellung von p-Hydroxy-benzaldehyden der Formel

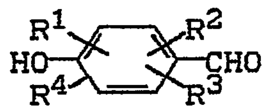

,

in der
$R^1$ bis $R^4$ unabhängig voneinander Wasserstoff, Halogen, $C_1$-$C_{10}$-Alkyl, $C_3$-$C_8$-Cycloalkyl, Phenyl oder $C_1$-$C_{10}$-Alkoxy bedeuten,
durch Oxidation von p-Kresolen der Formel

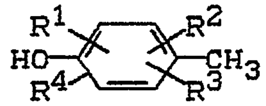

,

worin
$R^1$ bis $R^4$ die angegebene Bedeutung haben,
mit Sauerstoff in Gegenwart basisch reagierender Stoffe in einem Lösungsmittel, dadurch gekennzeichnet, daß in Gegenwart eines Chelatkomplexes von Eisen, Mangan oder Eisen und Mangan gearbeitet wird, wobei als Chelatbildner Porphyrine, Azaphorphzrine oder Phthalocyanine eingesetzt werden.

2. Verfahren nach Ansprüchen 1, dadurch gekennzeichnet, daß als Chelate Eisen-tetraaryl-porphine, Mangan-tetraaryl-porphine, Hämin, Eisenazaporphyrin, Eisen-phthalocyanin oder Eisen-phthalocyanin-tetrasulfonat eingesetzt werden.

3. Verfahren nach Ansprüchen 1 bis 2, dadurch gekennzeichnet, daß 0,000001-0,05 Mol Chelat, berechnet als Metall, pro Mol p-Kresol, bevorzugt 0,00001-0,005 eingesetzt werden.

4. Verfahren nach Ansprüchen 1 bis 3, dadurch gekennzeichnet, daß dem Chelatkomplex ein Co-Katalysator in Form einer Verbindung der Metalle Kupfer, Chrom, Mangan, Nickel, Eisen, Silber, Vanadium, Niob, Tantal, Cadmium, Cer oder weiterer Lanthaniden in einer Menge von 0,00001-0,1 Mol Metallverbindung, bevorzugt 0,0005-0,01 Mol Metallverbindung pro Mol p-Kresol zugesetzt wird.

5. Verfahren nach Ansprüchen 1 bis 4, dadurch gekennzeichnet, daß 0,001-1000 Mol Chelatkomplex, bevorzugt 0,01-100 Mol Chelatkomplex pro Mol Co-Katalysator eingesetzt werden.

6. Verfahren nach Anspruch 4, dadurch gekennzeichnet, daß anorganische Salze von Kupfer und/oder Cer als Co-Katalysator eingesetzt werden.

7. Verfahren nach Ansprüchen 1 bis 7, dadurch gekennzeichnet, daß in Gegenwart von Aktivkohle gearbeitet wird.

8. Verfahren nach Anspruch 1, dadurch gekennzeichnet, daß als Base ein Gemisch aus einem Alkali- oder Erdalkalihydroxid und einem Alkalialkoholat eingesetzt wird.

9. Verfahren nach Anspruch 8, dadurch gekennzeichent, daß das Äquivalentverhältnis von Alkali- oder Erdalkalihydroxid zu Alkalialkoholat 50:1 – 1:50, bevorzugt 8:1 – 1:4, besonders bevorzugt 4:1 – 2:3, beträgt.

## Claims

1. Process for the preparation of p-hydroxybenzaldehydes of the formula

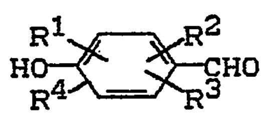

in which
$R^1$ to $R^4$ independently of one another denote hydrogen, halogen, $C_1$–$C_{10}$-alkyl, $C_3$–$C_8$-cycloalkyl, phenyl or $C_1$–$C_{10}$-alkoxy, by oxidizing p-cresols of the formula

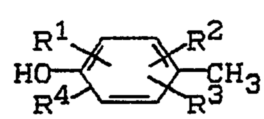

in which
$R^1$ to $R^4$ have the meaning given, with oxygen in the presence of basic substances in a solvent, characterized in that the oxidation is carried out in the presence of a chelate complex of iron, manganese or iron

and manganese, the chelating agents used being porphyrins, azoporphyrins or phthalocyanines.

2. Process according to Claim 1, characterized in that the chelates used are iron tetraarylporphins, manganese tetraarylporphins, haemine, iron azaporphyrin, iron phthalocyanine or iron phthalocyanine

3. Process according to Claims 1 to 2, characterized in that 0.000001–0.05 mol of chelate, calculated as metal, are used per mol of p-cresol, preferably 0.00001–0.005.

4. Process according to Claims 1 to 3, characterized in that a co-catalyst is added to the chelate complex in the form of a compound of the metals copper, chromium, manganese, nickel, iron, silver, vanadium, niobium, tantalum, cadmium, cerium or of further lanthanides in an amount of 0.00001–0.1 mol of metal compound, preferably 0.0005–0.01 mol of metal compound per mol of p-cresol.

5. Process according to Claims 1 to 4, characterized in that 0.001–1000 mol of chelate complex, preferably 0.01–100 mol of chelate complex are used per mol of cocatalyst.

6. Process according to Claim 4, characterized in that inorganic salts of copper and/or cerium are used as co-catalyst.

7. Process according to Claims 1 to 6, characterized in that the reaction is carried out in the presence of activated carbon.

8. Process according to Claim 1, characterized in that the base used is a mixture of an alkali metal hydroxide or alkaline earth metal hydroxide and an alkali metal alcoholate.

9. Process according to Claim 8, characterized in that the equivalent ratio of alkali metal hydroxide or alkaline earth metal hydroxide to alkali metal alcoholate is 50:1 – 1:50, preferably 8:1 – 1:4, particularly preferably 4:1 – 2:3.

**Revendications**

1. Procédé de production de p-hydroxybenzaldéhydes suivant la formule

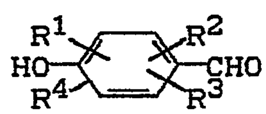

dans laquelle
$R^1$ à $R^4$ représentent, indépendamment les uns des autres, de l'hydrogène, un halogène, un radical alkyle en $C_1$–$C_{10}$, un radical cycloalkyle en $C_3$–$C_8$, un radical phényle ou un radical alcoxy en $C_1$–$C_{10}$, par oxydation des p-crésols suivant la formule

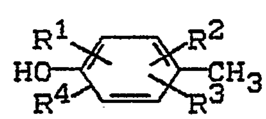

dans laquelle
$R^1$ à $R^4$ ont les significations précitées, par 1, oxygène en présence de substances à réaction basique dans un solvant, caractérisé en ce que l'on opère en présence d'un complexe chélaté de fer, de manganèse ou de fer et de manganèse, tandis que les agents chélatants utilisés sont des porphyrines, des azaporphyrines ou des phtalocyanines.

2. Procédé selon la revendication 1, caractérisé en ce que l'on utilise comme chélatesles tétra-arylporphines de fer, les tétra-arylporphines de manganèse, l'hémine, l'azaporphyrine de fer, la phtalocyanine de fer ou le tétrasulfonate de phtalocyanine de fer.

3. Procédé selon les revendications 1 à 2, caractérisé en ce que l'on utilise 0,000001 à 0,05 mole de chélate calculées sous forme de métal pour chaque mole de p-crésol et, de préférence, 0,00001 à 0,005.

4. Procédé selon les revendications 1 à 3, caractérisé en ce que l'on ajoute au complexe chélaté un cocatalyseur sous forme d'un composé des métaux cuivre, chrome, manganèse, nickel, fer, argent, vanadium, niobium, tantale, cadmium, cérium ou d'autres lanthanides, en quantités égales à 0,00001–0,1 mole de composé métallique et, de préférence, à 0,0005–0,01 mole de composé métallique par mole de p-crésol.

5. Procédé selon les revendications 1 à 4, caractérisé en ce que l'on utilise 0,001–1000 moles de complexe chélaté et, de préférence, 0,01–100 moles de complexe chélaté par mole de co-catalyseur.

6. Procédé selon la revendication 4, caractérisé en ce que l'on utilise des sels inorganiques de cuivre et/ou de cérium comme co-catalyseur.

7. Procédé selon les revendications 1 à 6, caractérisé en ce que l'on opère en présence de charbon actif.

8. Procédé selon la revendication 1, caractérisé en ce que l'on utilise comme base un mélange d'hydroxyde alcalin ou alcalino-terreux et un alcoolate alcalin.

9. Procédé selon la revendication 8, caractérisé en ce que le rapport équivalent de l'hydroxyde alcalin ou alcalino-terreux à l'alcoolate alcalin se situe dans le domaine allant de 50 : 1 à 1 : 50 et, de préférence, de 8 : 1 à 1 : 4 et, de préférence encore, de 4 : 1 à 2 : 3.